# EUROPEAN PATENT APPLICATION

(11) **EP 4 541 792 A1**
(43) Date of publication of application: **23.04.2025**
(21) Application number: 23823961.0
(22) Date of filing: 14.06.2023
(51) Int. Cl.: C07D 317/38

(54) **METHOD FOR PRODUCING ALKYLENE CARBONATE, AND APPARATUS FOR PRODUCING ALKYLENE CARBONATE**

(30) Priority: 15.06.2022 JP 2022096402
(71) Applicant: Asahi Kasei Kabushiki Kaisha, Tokyo 1000006 (JP)
(72) Inventor: OMURA, Shumpei, Tokyo 100-0006 (JP)
(74) Representative: Strehl & Partner mbB
(86) International application number: PCT/JP2023/022140
(87) International publication number: WO 2023/243671

(57) **Abstract**

A method for producing an alkylene carbonate using a first reaction vessel in which a reaction liquid containing a catalyst and carbon dioxide in a gaseous state are contained, comprising; a step (A) of feeding a raw material liquid containing an alkylene oxide through a nozzle so that the raw material liquid moves from the upper part of the first reaction vessel along the inner surface to the lower part thereof to feed the raw material liquid having dissolved carbon dioxide in the first reaction vessel to the reaction liquid; and a step (B) of reacting the alkylene oxide and carbon dioxide in the reaction liquid containing the catalyst in the lower part of the first reaction vessel to obtain the alkylene carbonate; wherein the ratio (DL/DT) of the height DL from the liquid level in the first reaction vessel to the discharge outlet of the nozzle to the height DT from the bottom tangent line of the first reaction vessel to the discharge outlet of the nozzle is 0.1 to 0.7.

## Description

### Technical Field

The present invention relates to a method for producing an alkylene carbonate and an apparatus for producing an alkylene carbonate.

### Background Art

Many studies have been made of the reaction for obtaining an alkylene carbonate from an alkylene oxide and carbon dioxide due to its usefulness, and active studies have been also made of catalysts for obtaining an industrially sufficient reaction rate. Many reports have been made on the use of solid acid catalysts, alkali metal salt catalysts, homogeneous organometallic catalysts and like. Examples thereof include a method for producing an alkylene carbonate using, as a catalyst, a carboxylic acid-type cation exchange resin with an alkyl group-substituted ammonium cation as a counter cation (Patent Literature 1); a method for producing an alkylene carbonate using a catalyst composed of tungsten oxide or molybdenum oxide (Patent Literature 2); a method for producing an alkylene carbonate using, as a catalyst, an anion exchange resin having a tertiary amine functional group or a quaternary ammonium functional group (Patent Literature 3); a synthesis of an aryl-substituted alkylene carbonate using an alkali metal salt as a catalyst (Patent Literature 4); a method for producing an alkenyl ether carbonate using a phase-transfer organometallic complex catalyst (Patent Literature 5); and a method for producing an alkylene carbonate from an alkylene oxide and carbon dioxide in the presence of an alkali halide catalyst (Patent Literature 6).

### Citation List

### Patent Literature

Patent Literature 1: Japanese Patent Laid-Open No. 7-206846
Patent Literature 2: Japanese Patent Laid-Open No. 7-206847
Patent Literature 3: Japanese Patent Laid-Open No. 7-206848
Patent Literature 4: Japanese Patent Laid-Open No. 8-53396
Patent Literature 5: U.S. Patent No. 5,095,124
Patent Literature 6: Japanese Patent Laid-Open No. 2006-104092

### Summary of Invention

### Technical Problem

The present invention aims to provide a method and an apparatus for producing an alkylene carbonate using an alkylene oxide and carbon dioxide as raw materials, in which a high conversion rate of an alkylene oxide is exhibited.

### Solution to Problem

After diligent research to solve the above problem, the present inventor has found that a reaction exhibiting a high conversion rate of an alkylene oxide can be achieved by setting, to a specified range, the ratio of the height DL from the liquid level in a reaction vessel to the discharge outlet of a nozzle for feeding a raw material liquid to the height DT from the bottom tangent line of the reaction vessel to the discharge outlet of the nozzle, which have led to the present invention.

The present invention includes the following embodiments:
<1>
   A method for producing an alkylene carbonate using a first reaction vessel in which a reaction liquid containing a catalyst and carbon dioxide in a gaseous state are contained, comprising;
   a step (A) of feeding a raw material liquid containing an alkylene oxide through a nozzle so that the raw material liquid moves from an upper part of the first reaction vessel along an inner surface to a lower part thereof to feed the raw material liquid having dissolved carbon dioxide in the first reaction vessel to the reaction liquid; and
   a step (B) of reacting the alkylene oxide and carbon dioxide in the reaction liquid containing the catalyst in a lower part of the first reaction vessel to obtain the alkylene carbonate;
   wherein a ratio (DL/DT) of a height DL from a liquid level in the first reaction vessel to a discharge outlet of the nozzle to a height DT from a bottom tangent line of the first reaction vessel to the discharge outlet of the nozzle is 0.1 to 0.7.
<2>
   The method for producing an alkylene carbonate according to <1>, wherein two or more of the nozzles are used.
<3>
   The method for producing an alkylene carbonate according to <1> or <2>, wherein:
   the reaction liquid contained in the first reaction vessel is drawn from a bottom part of the first reaction vessel, and a portion thereof is fed into the first reaction vessel as the raw material liquid, and
   a number of circulations of the reaction liquid per unit time is 10 to 70 /hr.
<4>
   The method for producing an alkylene carbonate according to any of <1> to <3>, wherein an operating pressure of the first reaction vessel is 4 to 12 MPa.
<5>
   The method for producing an alkylene carbonate according to any of <1> to <4>, wherein the ratio (DL/DT) is 0.20 to 0.60.
<6>
   The method for producing an alkylene carbonate according to any of <1> to <5>, wherein a height/inner diameter ratio of the first reaction vessel is 3 to 7, and the inner diameter is 1 to 4 m.
<7>
   The method for producing an alkylene carbonate according to any of <1> to <6>, wherein the alkylene oxide is ethylene oxide, and the alkylene carbonate is ethylene carbonate.
<8>
   The method for producing an alkylene carbonate according to any of <1> to <7>, comprising
   a step (C) of reacting the reaction liquid obtained in the step (B) with the alkylene oxide and carbon dioxide in a second reaction vessel to obtain the alkylene carbonate.
<9>
   The method for producing an alkylene carbonate according to <8>, comprising
   a step (D) of further reacting the reaction liquid obtained in the step (C) with the alkylene oxide and carbon dioxide in a third reaction vessel to obtain the alkylene carbonate.
<10>
   The method for producing an alkylene carbonate according to any of <1> to <9>, wherein in the step (A), the raw material liquid is jetted and fed against a wall surface through the nozzle.
<11>
   An apparatus for producing an alkylene carbonate, comprising:
   a first reaction vessel in which a reaction liquid containing a catalyst is contained;
   a nozzle for feeding a raw material liquid containing an alkylene oxide so that the raw material liquid moves from the upper part of the first reaction vessel along the inner surface to the lower part thereof; and
   a carbon dioxide feed section for introducing carbon dioxide into the first reaction vessel;
   wherein the apparatus is operated and controlled so that the ratio (DL/DT) of the height DL from the liquid level in the first reaction vessel to the discharge outlet of the nozzle to the height DT from the bottom tangent line of the first reaction vessel to the discharge outlet of the nozzle is 0.1 to 0.7.
<12>
   The apparatus for producing an alkylene carbonate according to <11>, further comprising:
   a second reaction vessel in which the reaction liquid containing the catalyst is contained;
   a nozzle for feeding the reaction liquid from the first reaction vessel as a raw material liquid so that it moves from the upper part of the second reaction vessel along the inner surface to the lower part thereof; and
   a carbon dioxide feed section for introducing carbon dioxide into the second reaction vessel.
<13>
   The apparatus for producing an alkylene carbonate according to <11> or <12>, further comprising a third reaction vessel into which the reaction liquid is introduced from the second reaction vessel and in which the alkylene oxide and carbon dioxide are further reacted.
<14>
   The apparatus for producing an alkylene carbonate according to any of <11> to <13>, wherein the ratio (DL/DT) is 0.10 to 0.40.
<15>
   The apparatus for producing an alkylene carbonate according to any of <11> to <14>, wherein the raw material liquid is jetted and fed against the wall surface.

### Advantageous Effects of Invention

The present invention can provide a method and an apparatus for producing an alkylene carbonate using an alkylene oxide and carbon dioxide as raw materials, in which a high conversion rate of an alkylene oxide is exhibited.

### Brief Description of Drawings

[Figure 1] Figure 1 is a schematic block diagram of an apparatus 1 for producing an alkylene carbonate according to a first embodiment.
[Figure 2] Figure 2 is a schematic block diagram of an apparatus 10 for producing an alkylene carbonate according to a second embodiment.

### Description of Embodiments

Hereinafter, embodiments of the present invention (hereinafter referred to as "the present embodiment") will be described in detail with reference to the figures as necessary, but the present invention is not limited thereto, and various variations can be made without departing from the scope of the present invention. In the figures, the positional relationships, such as the top and bottom, and left and right, are based on those shown in the figures, unless otherwise noted. Furthermore, the dimensional ratios in the figures are not limited to those shown therein.

The method for producing an alkylene carbonate according to the present embodiment, which uses a first reaction vessel in which a reaction liquid containing a catalyst and carbon dioxide in a gaseous state are contained, comprises:
a step (A) of feeding a raw material liquid containing an alkylene oxide through a nozzle so that the raw material liquid moves from the upper part of the first reaction vessel along the inner surface to the lower part thereof to feed the raw material liquid having dissolved carbon dioxide in the first reaction vessel to the reaction liquid; and
a step (B) of reacting the alkylene oxide and carbon dioxide in the reaction liquid containing the catalyst in the lower part of the first reaction vessel to obtain the alkylene carbonate;
wherein the ratio (DL/DT) of the height DL from the liquid level in the first reaction vessel to the discharge outlet of the nozzle to the height DT from the bottom tangent line of the first reaction vessel to the discharge outlet of the nozzle is 0.1 to 0.7.

The above configuration can provide a method for producing an alkylene carbonate using an alkylene oxide and carbon dioxide as raw materials, in which a high conversion rate of an alkylene oxide is exhibited. In the production method according to the present embodiment, a raw material liquid containing an alkylene oxide is fed through a nozzle so that the raw material liquid moves from the upper part of the reaction vessel along the inner surface to the lower part thereof, and thereby, the raw material liquid, which is spreading over the wall surface of the reaction vessel, absorbs carbon dioxide present in the reaction vessel, and it is fed to the reaction liquid in the lower part of the reaction vessel. This is considered to increase the concentration of carbon dioxide in the reaction liquid, making it easier to obtain the desired alkylene carbonate. In particular, it is presumed that by carrying out the operation with the ratio (DL/DT) in a specified range, the balance between the amount of carbon dioxide absorbed by the raw material liquid and the reaction efficiency is improved, making it possible to realize a reaction that exhibits a high conversion rate of an alkylene oxide. The above is a speculation on the mechanism by which the effects of the production method according to the present embodiment are obtained, and the present invention is not limited thereto.

The method for producing an alkylene carbonate according to the present embodiment may comprise, in addition to the steps (A) and (B),
a step (C) of reacting the reaction liquid obtained in the step (B) with the alkylene oxide and carbon dioxide in a second reaction vessel to obtain the alkylene carbonate.

The step (C) may comprise:
a step (C-1) of feeding the reaction liquid obtained in the step (B) as a raw material liquid trough a nozzle so that it moves from the upper part of the second reaction vessel along the inner surface to the lower part thereof in which the reaction liquid containing the catalyst and carbon dioxide in a gaseous state are contained to feed the raw material liquid having dissolved carbon dioxide in the second reaction vessel to the reaction liquid; and
a step (C-2) of reacting the alkylene oxide and carbon dioxide in the reaction liquid containing the catalyst in the lower part of the second reaction vessel to obtain the alkylene carbonate.

The method for producing an alkylene carbonate according to the present embodiment may comprise, in addition to the steps (A), (B) and (C):
a step (D) of further reacting the reaction liquid obtained in the step (C) with the alkylene oxide and carbon dioxide in a third reaction vessel to obtain the alkylene carbonate.
The method for producing an alkylene carbonate according to the present embodiment can further increase the conversion rate of an alkylene oxide by including the above configuration.

### <Raw material liquid>

The raw material liquid contains an alkylene oxide. Examples of the alkylene oxide include a compound represented by the following formula (I): 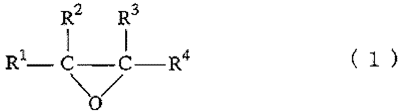 wherein R¹, R², R³ and R⁴ each independently represent a hydrogen atom, a linear hydrocarbon group having 1 to 8 carbon atoms, an alicyclic hydrocarbon group having 3 to 8 carbon atoms, or an aromatic hydrocarbon group having 6 to 8 carbon atoms.

Specific examples of the alkylene oxide include ethylene oxide, propylene oxide, butylene oxide, vinyl ethylene oxide, cyclohexene oxide and styrene oxide. Among these, ethylene oxide and propylene oxide are preferred from the viewpoint of availability.

In the method for producing an alkylene carbonate according to the present embodiment, in a step of obtaining an alkylene carbonate, an alkylene oxide is reacted with carbon dioxide in the presence of a catalyst to obtain an alkylene carbonate (a cyclic alkylene carbonate) represented by the following formula (2): 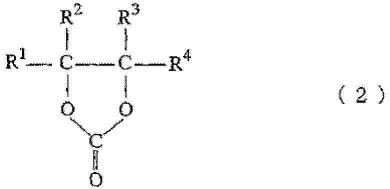 wherein R¹, R², R³ and R⁴ are as defined in formula (1) .

In the present embodiment, specific examples of the alkylene carbonate include, but not particularly limited to, ethylene carbonate, propylene carbonate, butylene carbonate, vinyl ethylene carbonate, cyclohexene carbonate and styrene carbonate, and ethylene carbonate and propylene carbonate are preferred.

In the method for producing an alkylene carbonate, a reaction for obtaining the alkylene carbonate from an alkylene oxide and carbon dioxide is represented by the following reaction formula (3): 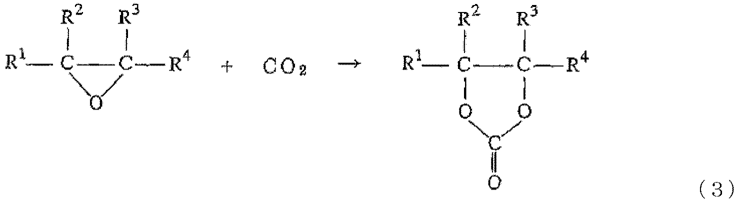 wherein R¹, R², R³ and R⁴ are as defined in formula (1).

The catalyst used in the above reaction is not particularly limited as long as it is a catalyst to be used for carrying out the reaction of the above reaction formula (3). Specific examples of the catalyst to be used in the above reaction include an organic catalyst such as tetraethylammonium bromide, a halide of a 5- or 6-membered ring hydrocarbon, ammonium rhodanide or a thermal decomposition product thereof; an inorganic catalyst such as a metal or alkali metal bromide or iodide; and those to which a small amount of an alcohol or water is added, but an inorganic catalyst that is easy to recover is preferred. The amount of catalyst to be used is not particularly limited, and is preferably 0.1 to 3% by mass, and more preferably 0.1 to 2% by mass, relative to the raw material liquid.

### [First embodiment]

### <Apparatus for producing alkylene carbonate>

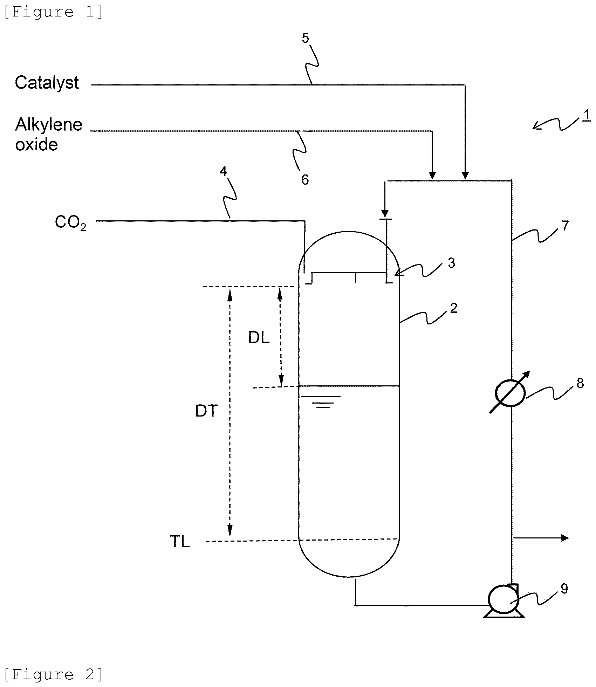

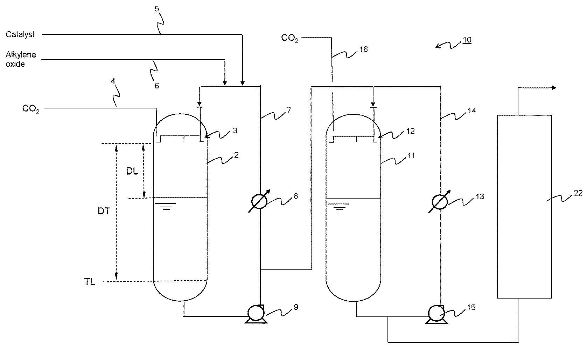

Figure 1 is a schematic block diagram of an apparatus 1 for producing an alkylene carbonate according to a first embodiment. The apparatus 1 for producing an alkylene carbonate according to the present embodiment comprises a first reaction vessel 2; a nozzle 3 for feeding a raw material liquid; and a carbon dioxide feed section 4 for introducing carbon dioxide.

The first reaction vessel 2 may be, for example, such a reaction system as a complete mixing reactor. The complete mixing reactor may be equipped with a Venturi stirring bar.

A reaction liquid containing a catalyst is contained in the first reaction vessel 2. The reaction liquid may be retained in the lower part of the first reaction vessel 2 and may be stirred with a stirrer or the like (not shown). Carbon dioxide is introduced into the first reaction vessel 2 from the carbon dioxide feed section 4. Gas-phase carbon dioxide is present in a high concentration in the upper part of the first reaction vessel 2.

The nozzle 3 preferably has a structure suitable for dissolving carbon dioxide in the raw material liquid, and examples thereof include a shower nozzle.

The nozzle 3 may be connected to a catalyst feed section 5 and an alkylene oxide feed section 6 to be configured to feed the raw material liquid containing a mixture of the catalyst and ethylene oxide. The catalyst is adjusted to a specified concentration before being mixed with the raw material liquid.

The nozzle 3 is installed at the upper part of the first reaction vessel 2 so that the raw material liquid is fed to the inner wall surface of the first reaction vessel 2. Two or more nozzles 3 are preferably provided. By providing multiple nozzles 3, the raw material liquid can spread over the inner surface of the reaction vessel to increase the absorption efficiency of carbon dioxide.

The height/inner diameter ratio of the reaction vessel is preferably 3 to 7, more preferably 4 to 6 and even more preferably 4.5 to 5.5. As used herein, the height of the reaction vessel refers to the distance between the tangent lines at the upper and lower parts.

The inner diameter of the reaction vessel is preferably 1 to 4 m, more preferably 1.2 to 3.0 m and even more preferably 1.5 to 2.5 m.

As shown in Figure 1, the apparatus for producing an alkylene carbonate preferably has a circulation circuit 7 for reusing the reaction liquid from the first reaction vessel 2 as the raw material liquid. The circulation circuit 7 is equipped with an external pump 9 and is configured to be able to circulate the reaction liquid. The external pump 9 may be provided in combination with a spare external pump (not shown). The circulation circuit 7 preferably communicates with a process side flow path of a heat exchanger 8 via piping. The heat exchanger 8 preferably has a heat exchange side flow path for flowing a heat exchange medium having the temperature adjusted within a specified temperature range, and a process side flow path for flowing a process liquid in which heat exchange is carried out in association with the production of the alkylene carbonate. The process liquid is a liquid which is treated (i.e. the temperature of which is adjusted) by the heat exchanger, and the heat exchange medium is a medium for adjusting the temperature of the process liquid. The heat exchange side flow path of the heat exchanger is a flow path for flowing the heat exchange medium, and the process side flow path is a flow path for flowing the process liquid.

The heat exchanger 8 is preferably a heat exchanger that can flow the heat exchange medium having a temperature of 140°C to 200°C through the heat exchange side flow path thereof and maintain the internal temperature of the process side flow path at 135°C to 200°C. For example, a coiled heat exchanger to be provided inside the reactor, a double-pipe heat exchanger, a general multiple-pipe heat exchanger or the like can be used alone or in combination. A multiple-pipe heat exchanger, which has a larger heat transfer area and can downsize the apparatus, is preferably used.

When the multiple-pipe heat exchanger is used, the process liquid and the heat exchange medium can be passed through either the tube side or the shell side of the multiple-pipe heat exchanger as the heat exchange side flow path or the process side flow path. The liquid to be passed through each of the tube side and the shell side of the heat exchanger may be selected as necessary, such as when increasing the overall heat transfer coefficient (U) to use a small heat exchanger, or when passing a fluid, to which contaminants easily adhere, through the tube side to facilitate cleaning.

The heat exchanger 8 is preferably a device that functions as both a preheater and a cooler. Such a heat exchanger can be used as a preheater to increase the temperature of the reaction liquid to the reaction starting temperature during start-up, and as a cooler to remove the heat of reaction during steady operation.

The material of the process side flow path of the heat exchanger 8 is not particularly limited as long as it has corrosion resistance to the process liquid. Stainless steel is preferably used because iron rust causes the formation of alkylene oxide polymers due to its catalytic action.

### <Method for producing alkylene carbonate>

Hereinafter, the method for producing an alkylene carbonate according to the present embodiment will be described, taking, as an example, an operation mode using the apparatus 1 for producing an alkylene carbonate.

A catalyst and an alkylene oxide are fed from the catalyst feed section 5 and the alkylene oxide feed section 6, respectively to obtain a raw material liquid containing these. Here, the amount of catalyst to be fed may be adjusted to obtain a raw material liquid having a desired catalyst concentration.

Subsequently, the nozzle 3 feeds the raw material liquid so that it moves from the upper part of the first reaction vessel 2 along the inner surface to the lower part thereof. As the raw material liquid moves along the inner surface, it spreads out flat and forms a liquid film, so that gas-phase carbon dioxide is dissolved in the raw material liquid. The raw material liquid having carbon dioxide dissolved flows into and is mixed with a reaction liquid containing the catalyst, thereby feeding the alkylene oxide and carbon dioxide to the reaction liquid. By the above operation, the above-described step (A) is carried out.

In the step (A), the raw material liquid is preferably jetted and fed against the wall surface through the nozzle 3. By adjusting the direction of the nozzle 3 in such a way, the raw material liquid can move along the inner surface of the reactor.

The ratio of the distance from a jetting outlet of the nozzle 3 to the wall surface to the inner diameter of the reactor (the distance of the jetting outlet of the nozzle 3 - the wall surface/the inner diameter of reactor) is preferably 0.30 or less, more preferably 0.20 or less, and even more preferably 0.10 or less. The distance from the jetting outlet of the nozzle 3 to the wall surface refers to the distance from the jetting outlet of the nozzle 3 to the wall surface in the direction of the raw material liquid-jetting axis of the nozzle. For example, if the jetting outlet of the nozzle 3 is not facing the wall surface, the distance from the jetting outlet to the wall surface cannot be defined.

As shown in Figure 1, in the method for producing an alkylene carbonate according to the present embodiment, the ratio (DL/DT) of the height DL from the liquid level in the reaction vessel to the discharge outlet of the nozzle to the height DT from the bottom tangent line of the first reaction vessel 2 to the discharge outlet of the nozzle is 0.1 to 0.7. The ratio (DL/DT) is preferably 0.10 to 0.60, more preferably 0.10 to 0.50, even more preferably 0.10 to 0.45, and still more preferably 0.10 to 0.40. The smaller the value of the ratio (DL/DT) is, the smaller the space above the first reaction vessel 2 is, making it more difficult to absorb carbon dioxide. However, in the present invention, since the raw material liquid is fed through the nozzle so that it moves from the upper part of the first reaction vessel along the inner surface to the lower part thereof, the residence time is ensured in the space at the upper part and a sufficient amount of carbon dioxide is thereby absorbed into the raw material liquid. On the other hand, the smaller the value of the ratio (DL/DT) is, the higher the liquid level in the first reaction vessel 2 is. Therefore, by keeping the ratio (DL/DT) within the above-described range, sufficient reaction time can be ensured after the raw material liquid is introduced into the liquid phase, resulting in a high conversion rate of the alkylene oxide.

The bottom tangent line refers to the height of the boundary between the straight body part and the curved surface of the reaction vessel.

When multiple nozzles 3 are provided, DT is the average value of the height from the bottom tangent line of the first reaction vessel 2 to the discharge outlet of each nozzle, and DL is the average value of the height from the liquid level in the reaction vessel to the discharge outlet of each nozzle.

The production apparatus according to the present embodiment is operated and controlled so that the ratio (DL/DT) is 0.1 to 0.7, which can be carried out, for example, by cascade control based on the liquid level in the reactor as measured by a level gauge and the withdrawal flow rate of the reactor.

The above-described step (B) is carried out in the reaction liquid in the lower part of the first reaction vessel 2.

The reaction temperature in the step (B) is preferably 100 to 250°C, more preferably 150 to 200°C, and even more preferably 160 to 190°C.

The average residence time in the reaction vessel in the step (B) is preferably 10 minutes to 6 hours, more preferably 30 minutes to 3 hours, and more preferably 1 to 2 hours.

The operating pressure of the first reaction vessel 2 is preferably 4 to 12 MPa, more preferably 5 to 11 MPa, and even more preferably 8 to 10 MPa.

It is preferred that the reaction liquid contained in the first reaction vessel 2 is withdrawn from the bottom part of the first reaction vessel 2, and a portion thereof is fed into the first reaction vessel 2 as a raw material liquid. That is, the reaction liquid is preferably circulated as a raw material liquid via the circulation circuit 7 and the external pump 9.

In this case, the number of circulations of the reaction liquid per unit time is preferably 10 to 70 /hr, and more preferably 20 to 50 /hr. The number of circulations refers to the number of replacements of the reaction liquid in the reaction vessel per hour, and is expressed by the following equation (A): Number of circulations = Circulation flow rate by external pump/Volume of liquid phase at lower part of reactor (A)

In case that the reaction liquid is circulated, if a heat exchanger is provided in the middle of piping to remove the heat of reaction, the reaction liquid is preferably circulated at a high flow rate because the cooling capacity of the heat exchanger is increased.

The method for producing an alkylene carbonate according to the present embodiment is preferably carried out on an industrial scale. As used herein, the industrial scale refers to a scale on which an alkylene carbonate is produced at a rate of 1 t (ton)/hour or more, preferably 2 t (tons)/hour or more, more preferably 3 t (tons)/hour or more, and even more preferably 4 t (tons)/hour or more. The upper limit of the industrial scale is not particularly limited, and is, for example, 15 t (tons)/hour or less.

### [Second embodiment]

Figure 2 is a schematic block diagram of an apparatus 10 for producing an alkylene carbonate according to a second embodiment. The same symbols apply to the components that are common to the apparatus 1 for producing an alkylene carbonate and the description of the same components is omitted. The components characteristic of the apparatus 10 for producing an alkylene carbonate will be described below. The apparatus 10 for producing an alkylene carbonate further has a second reaction vessel 11 and a third reaction vessel 22 downstream of the first reaction vessel 2.

The apparatus 10 for producing an alkylene carbonate according to the present embodiment comprises the second reaction vessel 11 in addition to the components shown in the apparatus 1 for producing an alkylene carbonate. A reaction liquid containing a catalyst is contained in the second reaction vessel 11. The second reaction vessel 11 comprises a nozzle 12 for feeding the reaction liquid from the first reaction vessel 2 as a raw material liquid so that it moves from the upper part of the second reaction vessel 11 along the inner surface to the lower part thereof; and a carbon dioxide feed section 16 for introducing carbon dioxide into the second reaction vessel 11.

In the second reaction vessel 11, a step is carried out in which the reaction liquid is fed from the first reaction vessel 2 through a nozzle so that it moves from the upper part of the second reaction vessel 11 along the inner surface to the lower part thereof, and a raw material liquid having dissolved carbon dioxide in the second reaction vessel 11 is fed to the reaction liquid, thereby allowing the reaction between the alkylene oxide and carbon dioxide to further proceed. By the above operation, the above-described step (C) (steps (C-1) and (C-2)) is carried out. In the second reactor 11, carbon dioxide may be fed from the carbon dioxide feed section 16, but a sufficient amount of carbon dioxide is dissolved in the reaction liquid in the first reactor 2. Therefore, if the carbon dioxide concentration in the reaction liquid becomes low, carbon dioxide may be fed appropriately from the carbon dioxide feed section 16.

In the method for producing an alkylene carbonate according to the present embodiment, the ratio (DL'/DT') of the height DL' from the liquid level in the second reaction vessel 11 to the discharge outlet of the nozzle 12 to the height DT' from the bottom tangent line of the second reaction vessel 11 to the discharge outlet of the nozzle 12 is preferably 0.1 to 0.7, more preferably 0.15 to 0.60, and even more preferably 0.20 to 0.50.

When multiple nozzles 12 are provided, DT' is the average value of the height from the bottom tangent line of the second reaction vessel 11 to the discharge outlet of each nozzle, and DL' is the average value of the height from the liquid level in the reaction vessel to the discharge outlet of each nozzle.

As shown in Figure 2, the apparatus 10 for producing an alkylene carbonate preferably has a circulation circuit 14 for reusing the reaction liquid from the second reaction vessel 11 as a raw material liquid. The circulation circuit 14 may have a heat exchanger 13 and an external pump 15. The external pump 15 may be provided in combination with a spare external pump (not shown). The circulation circuit 14 preferably communicates with a process side flow path of the heat exchanger 13 via piping. By providing the circulation circuit 14, a portion of the excess carbon dioxide is released from the second reaction vessel 11.

The reaction temperature in the second reaction vessel 11 is preferably 100 to 250°C, more preferably 150 to 200°C, and even more preferably 160 to 190°C.

The average residence time in the second reaction vessel 11 is preferably 10 minutes to 6 hours, more preferably 30 minutes to 3 hours, and more preferably 2 to 3 hours.

The operating pressure of the second reaction vessel 11 is preferably 4 to 12 MPa, more preferably 4 to 8 MPa, and even more preferably 4.5 to 6.5 MPa.

It is preferred that the reaction liquid contained in the second reaction vessel 11 is withdrawn from the bottom part of the second reaction vessel 11, and a portion thereof is fed into the second reaction vessel 11 as a raw material liquid. That is, the reaction liquid is preferably circulated as a raw material liquid via the circulation circuit 14 and the external pump 15.

In this case, the number of circulations of the reaction liquid per unit time is preferably 10 to 70 /hr, and more preferably 15 to 35 /hr.

The apparatus 10 for producing an alkylene carbonate according to the present embodiment comprises a third reaction vessel 22, downstream of the second reaction vessel 11, into which the reaction liquid is introduced from the second reaction vessel 11 and in which the alkylene oxide and carbon dioxide are further reacted. In the third reaction vessel 22, the reaction between the unreacted alkylene oxide and the dissolved carbon dioxide is carried out. The reaction liquid obtained in the step (C) is further reacted with the alkylene oxide and carbon dioxide in the third reaction vessel 22 to obtain the alkylene carbonate. By the above operation, the above-described step (D) is carried out. The third reaction vessel 22 is preferably a flooded reactor such as a plug-flow reactor with no external circulation.

The reaction temperature in the third reaction vessel 22 is preferably 100 to 250°C, more preferably 150 to 200°C, and even more preferably 160 to 190°C.

The operating pressure of the third reaction vessel 22 is preferably 4 to 12 MPa, more preferably 4 to 8 MPa, and even more preferably 4.5 to 6.5 MPa.

### Examples

Hereinafter, the present embodiments will be further specifically described with reference to the examples thereof, but the present embodiments will not be limited to the following examples.

### [Example 1]

The apparatus 10 for producing an alkylene carbonate shown in Figure 2 was used to produce ethylene carbonate. The first reaction vessel 2 is a stainless steel vertical cylindrical tank having an inner diameter of 1.8 mφ, a straight body length of 8.7 m and a volume of 23.6 m³. A distributor with 10 nozzles 3 is positioned, at the upper part of the first reaction vessel 2, 8.4 m (DT) above the tangent line at the lower part of the first reaction vessel 2 for the purpose of liquid dispersion to increase the absorption efficiency of carbon dioxide gas. The first reaction vessel 2 communicates with a process side flow path of the heat exchanger 8 via piping to form the circulation circuit 7.

Ethylene oxide cooled to about 5°C was fed as one of raw materials from the alkylene oxide feed section 6 to the first reaction vessel 2 at 4,780 kg/hr. As for another raw material, carbon dioxide, liquefied carbon dioxide was gasified in a carbon dioxide evaporator (not shown) and fed from the carbon dioxide feed section 4 into the first reaction vessel 2 while adjusting it to achieve a constant pressure of about 9.5 MPa at a temperature of about 90°C. The average feed rate of carbon dioxide was 4,920 kg/hr.

Potassium iodide (KI) was used as a catalyst, and was blended to be 5% by mass in an ethylene carbonate solution. The catalyst solution was prepared by blending 9 parts by mass of the recovered catalyst, which had been recovered after purifying an ethylene carbonate product, and 1 part by mass of a fresh catalyst solution, and was fed through the nozzle 3 to the wall surface of the first reaction vessel 2. The feed rate of the catalyst solution was set to 560 kg/hr with a pump 3 so that the concentration of potassium iodide in a circulating liquid (reaction system) was 0.23 to 0.26% by mass.

The reaction mixture was discharged from the first reaction vessel 2 so that the distance (DL) from the liquid level in the first reaction vessel 2 to the nozzle 3 was constant at 2.9 m. The discharge amount of the reaction mixture was adjusted with a feed control valve (not shown). (Ratio (DL/DT) = 0.35)

Ethylene oxide was produced under conditions that the reaction temperature was 180°C as measured with a thermometer provided at the bottom part of the first reaction vessel 2. During the reaction, the reaction liquid was flowed into the circulation circuit 7 including the first reaction vessel 2 and the process side flow path of the heat exchanger 8, while flowing a heat exchange medium into a heat exchange side flow path of the heat exchanger 8. Specifically, the reaction liquid was withdrawn from the outlet of the first reaction vessel 2, pressurized by the external pump 9, and fed to the heat exchanger 8. The reaction mixture, the temperature of which had been adjusted by the heat exchanger 8, was then returned from the inlet of the heat exchanger 8 to the inlet of the reaction vessel 2 to be circulated within the circulation circuit. The circulation rate of the reaction liquid in the first reaction vessel 2 was monitored by a circulation flow meter (not shown) and adjusted to be constant at about 550 tons/hr. The liquid retention volume was 17.5 tons, the average residence time in the first reaction vessel 2 was 1.7 hours, and the number of circulations was 44 /hr.

The reaction liquid withdrawn from the circulation circuit 7 of the first reaction vessel 2 was fed into the second reaction vessel 11. The second reaction vessel 11 is a stainless steel vertical cylindrical tank having an inner diameter of 2.3 mφ, a straight body length of 6.5 m and a volume of 33.4 m³. A distributor with 10 nozzles is positioned at the upper part of the second reaction vessel 11, 6.2 m above the tangent line at the lower part of the reaction vessel for the purpose of liquid dispersion to increase the absorption efficiency of carbon dioxide gas. The second reaction vessel 11 communicates with the process side flow path of the heat exchanger 13 via piping to form the circulation circuit 14. The circulation rate was adjusted to be constant at about 405 tons/hr. The reaction temperature was controlled at 180°C as measured by a thermometer provided at the bottom part of the second reaction vessel 11, and the reaction pressure was controlled at 5.1 MPa. The reaction mixture was discharged from the second reaction vessel 11 so that the distance from the liquid level in the second reaction vessel 11 to the distributor of the nozzle 12 was constant at 3.1 m. (Ratio (DL'/DT') = 0.50)

The reaction liquid withdrawn from the circulation circuit 14 of second reaction vessel 11 was fed into the third reaction vessel 22. The third reaction vessel 22 is a plug-flow reactor having an inner diameter of 0.9 mφ, a straight body length of 9.0 m and a volume of 6.1 m³. The reaction temperature was controlled at 180°C as measured by a thermometer provided at the bottom part of the third reaction vessel 22, and the reaction pressure was controlled at 5.1 MPa.

The operation was continuously carried out under the above conditions for 180 days, and stable production results were achieved.

The yield of ethylene carbonate at the outlet of the third reaction vessel 22 was 8,930 kg/hr, and the conversion rate of ethylene oxide to ethylene carbonate at the outlet of the first reaction vessel 2 was 88.6%. The conversion rate of ethylene oxide to ethylene carbonate at the outlet of the third reaction vessel 22 was 99.3%.

### [Example 2]

Ethylene carbonate was produced in the same manner as in Example 1, except that the distance (DT) from the liquid level in the first reaction vessel 2 to the feed position of the nozzle 3 was set to 4.2 m. (Ratio (DL/DT) = 0.50) The liquid retention volume was 15.4 tons, the average residence time in the first reaction vessel 2 was 1.4 hours, and the number of circulations was 52 /hr.

The operation was continuously carried out under the above conditions for 180 days, and stable production results were achieved. The yield of ethylene carbonate at the outlet of the third reaction vessel 22 was 8,730 kg/hr, and the conversion rate of ethylene oxide to ethylene carbonate at the outlet of the first reaction vessel 2 was 86.5%. The conversion rate of ethylene oxide to ethylene carbonate at the outlet of the third reaction vessel 22 was 99.2%.

### [Example 3]

Ethylene carbonate was produced in the same manner as in Example 1, except that the distance (DT) from the liquid level in the first reaction vessel 2 to the feed position of the nozzle 3 was set to 5.5 m. (Ratio (DL/DT) = 0.65) The liquid retention volume was 11.0 tons, the average residence time in the reaction vessel was 1.0 hours, and the number of circulations was 70 /hr.

The operation was continuously carried out under the above conditions for 180 days, and stable production results were achieved. The yield of ethylene carbonate at the outlet of the third reaction vessel 22 was 8,531 kg/hr, and the conversion rate of ethylene oxide to ethylene carbonate at the outlet of the first reaction vessel 2 was 84.5%. The conversion rate of ethylene oxide to ethylene carbonate at the outlet of the third reaction vessel 22 was 99.1%.

### [Example 4]

Ethylene carbonate was produced in the same manner as in Example 1, except that the number of nozzles 3 in the first reaction vessel 2 was one. The yield of ethylene carbonate at the outlet of the third reaction vessel 22 was 8,440 kg/hr, and the conversion rate of ethylene oxide to ethylene carbonate at the outlet of the first reaction vessel 2 was 83.5%. The conversion rate of ethylene oxide to ethylene carbonate at the outlet of the third reaction vessel 22 was 99.0%.

### [Comparative Example 1]

Ethylene carbonate was produced in the same manner as in Example 1, except that the distance (DT) from the liquid level in the first reaction vessel 2 to the feed position of the nozzle 3 was set to 0.2 m. (Ratio (DL/DT) = 0.02) The liquid retention volume was 27.5 tons, the average residence time in the reaction vessel was 2.7 hours, and the number of circulations was 28 /hr.

The yield of ethylene carbonate at the outlet of the third reaction vessel 22 was 8,330 kg/hr, and the conversion rate of ethylene oxide to ethylene carbonate at the outlet of the first reaction vessel 2 was 82.4%. The conversion rate of ethylene oxide to ethylene carbonate at the outlet of the third reaction vessel 22 was 98.9%.

### [Comparative Example 2]

Ethylene carbonate was produced in the same manner as in Example 1, except that the distance (DT) from the liquid level in the first reaction vessel 2 to the feed position of the nozzle 3 was set to 6.7 m. (Ratio (DL/DT) = 0.80) The liquid retention volume was 7.1 tons, the average residence time in the first reaction vessel 2 was 0.7 hours, and the number of circulations was 108 /hr. The yield of ethylene carbonate at the outlet of the third reaction vessel 22 was 7,840 kg/hr, and the conversion rate of ethylene oxide to ethylene carbonate at the outlet of the first reaction vessel 2 was 77.3%. The conversion rate of ethylene oxide to ethylene carbonate at the outlet of the third reaction vessel 22 was 99.0%.

### Industrial Applicability

The present invention can be suitably utilized as a method for producing an alkylene carbonate, which is useful as a solvent, a raw material for organic synthesis, and an electrolyte for secondary batteries, and as an apparatus to be used for the method.

### Reference Sings List

1,10: Apparatus for producing an alkylene carbonate
2: First reaction vessel
3,12: Nozzle
4,16: Carbon dioxide feed section
5: Catalyst feed section
6: Alkylene oxide feed section
7,14: Circulation circuit
8,13: Heat exchanger
9,15: External pump
11: Second reaction vessel
22: Third reaction vessel

## Claims

1. A method for producing an alkylene carbonate using a first reaction vessel in which a reaction liquid containing a catalyst and carbon dioxide in a gaseous state are contained, comprising;
a step (A) of feeding a raw material liquid containing an alkylene oxide through a nozzle so that the raw material liquid moves from an upper part of the first reaction vessel along an inner surface to a lower part thereof to feed the raw material liquid having dissolved carbon dioxide in the first reaction vessel to the reaction liquid; and
a step (B) of reacting the alkylene oxide and carbon dioxide in the reaction liquid containing the catalyst in the lower part of the first reaction vessel to obtain the alkylene carbonate;
wherein a ratio (DL/DT) of a height DL from a liquid level in the first reaction vessel to a discharge outlet of the nozzle to a height DT from a bottom tangent line of the first reaction vessel to the discharge outlet of the nozzle is 0.1 to 0.7.

2. The method for producing an alkylene carbonate according to claim 1, wherein two or more of nozzles are used.

3. The method for producing an alkylene carbonate according to claim 1, wherein:
the reaction liquid contained in the first reaction vessel is drawn from a bottom part of the first reaction vessel, and a portion thereof is fed into the first reaction vessel as the raw material liquid; and
a number of circulations of the reaction liquid per unit time is 10 to 70 /hr.

4. The method for producing an alkylene carbonate according to claim 1, wherein an operating pressure of the first reaction vessel is 4 to 12 MPa.

5. The method for producing an alkylene carbonate according to claim 1, wherein the ratio (DL/DT) is 0.10 to 0.40.

6. The method for producing an alkylene carbonate according to claim 1, wherein a height/inner diameter ratio of the first reaction vessel is 3 to 7, and the inner diameter is 1 to 4 m.

7. The method for producing an alkylene carbonate according to claim 1, wherein the alkylene oxide is ethylene oxide, and the alkylene carbonate is ethylene carbonate.

8. The method for producing an alkylene carbonate according to claim 1, comprising
a step (C) of reacting the reaction liquid obtained in the step (B) with the alkylene oxide and carbon dioxide in a second reaction vessel to obtain the alkylene carbonate.

9. The method for producing an alkylene carbonate according to claim 8, comprising
a step (D) of further reacting the reaction liquid obtained in the step (C) with the alkylene oxide and carbon dioxide in a third reaction vessel to obtain the alkylene carbonate.

10. The method for producing an alkylene carbonate according to any of claims 1 to 9, wherein in the step (A), the raw material liquid is jetted and fed against a wall surface through the nozzle.

11. An apparatus for producing an alkylene carbonate, comprising:
a first reaction vessel in which a reaction liquid containing a catalyst is contained;
a nozzle for feeding a raw material liquid containing an alkylene oxide so that the raw material liquid moves from the upper part of the first reaction vessel along the inner surface to the lower part thereof; and
a carbon dioxide feed section for introducing carbon dioxide into the first reaction vessel;
wherein the apparatus is operated and controlled so that the ratio (DL/DT) of the height DL from the liquid level in the first reaction vessel to the discharge outlet of the nozzle to the height DT from the bottom tangent line of the first reaction vessel to the discharge outlet of the nozzle is 0.1 to 0.7.

12. The apparatus for producing an alkylene carbonate according to claim 11, further comprising:
a second reaction vessel in which the reaction liquid containing the catalyst is contained;
a nozzle for feeding the reaction liquid from the first reaction vessel as a raw material liquid so that it moves from the upper part of the second reaction vessel along the inner surface to the lower part thereof; and
a carbon dioxide feed section for introducing carbon dioxide into the second reaction vessel.

13. The apparatus for producing an alkylene carbonate according to claim 12, further comprising a third reaction vessel into which the reaction liquid is introduced from the second reaction vessel and in which the alkylene oxide and carbon dioxide are further reacted.

14. The apparatus for producing an alkylene carbonate according to any of claims 11 to 13, wherein the ratio (DL/DT) is 0.10 to 0.40.

15. The apparatus for producing an alkylene carbonate according to any of claims 11 to 13, wherein the raw material liquid is jetted and fed against a wall surface through the nozzle.
